# EUROPEAN PATENT SPECIFICATION

(11) **EP 2 032 167 B1**
(45) Date of publication and mention of the grant of the patent: **17.08.2016**
(21) Application number: 07785840.5
(22) Date of filing: 22.06.2007
(51) Int. Cl.: A61K 39/395, A61P 31/16, A61P 37/06, C07K 16/28

(54) **A NOVEL TARGET IN THE TREATMENT OF CYTOKINE RELEASE SYNDROME**
NEUES TARGET BEI DER BEHANDLUNG DES ZYTOKIN-FREISETZUNGSYNDROMS
NOUVELLE CIBLE DANS LE TRAITEMENT DU SYNDROME DE LIBERATION DE CYTOKINES (CRS)

(30) Priority: 22.06.2006 EP 06012891
(43) Date of publication of application: 11.03.2009
(73) Proprietor: CellAct Pharma GmbH, 44227 Dortmund (DE)
(72) Inventor: UTKU, Nalan, 44227 Dortmund (DE)
(74) Representative: Krauss, Jan
(86) International application number: PCT/EP2007/005533
(87) International publication number: WO 2007/147623

(56) References cited:
- WO-A-03/025000
- WO-A-03/054018
- WO-A-03/054019
- UTKU N ET AL: "Antibody targeting of TIRC7 results in significant therapeutic effects on collagen-induced arthritis in mice" CLINICAL AND EXPERIMENTAL IMMUNOLOGY, vol. 144, no. 1, April 2006 (2006-04), pages 142-151, XP008085575 ISSN: 0009-9104
- UTKU NALÂN ET AL: "TIRC7 deficiency causes in vitro and in vivo augmentation of T and B cell activation and cytokine response." JOURNAL OF IMMUNOLOGY (BALTIMORE, MD. : 1950) 15 AUG 2004, vol. 173, no. 4, 15 August 2004 (2004-08-15), pages 2342-2352, XP002457830 ISSN: 0022-1767
- UTKU N ET AL: "PREVENTION OF ACUTE ALLOGRAFT REJECTION BY ANTIBODY TARGETING OF TIRC7, A NOVEL T CELL MEMBRANE PROTEIN" IMMUNITY, CELL PRESS, US, vol. 9, October 1998 (1998-10), pages 509-518, XP002188110 ISSN: 1074-7613
- GRUNEWALD SUSANNE M ET AL: "Infection with influenza A virus leads to flu antigen-induced cutaneous anaphylaxis in mice" JOURNAL OF INVESTIGATIVE DERMATOLOGY, vol. 118, no. 4, April 2002 (2002-04), pages 645-651, XP002457831 ISSN: 0022-202X

## Description

The present invention relates to means for the treatment of Cytokine Release Syndrome (CRS). In particular, the present invention concerns novel agents in the treatment and diagnosis of CRS. More specifically, the present invention discloses that T-cell immune response cDNA7 (TIRC7) protein is an important novel target for treating CRS, in particular when evoked with infection of pathogens or being due to drug side effects.

### Background of the invention

Human monocytes and murine macrophages are susceptible to infection by influenza A virus; see, e.g., review by Peschke et al., Immunobiology. 189 (1993), 340-355. Although virus replication is usually low, infection leads to cell death which is characterized by an extreme intracellular vacuolization. Most importantly, influenza A virus infection is accompanied by a particular pattern of cytokine release. Whereas IL-1 beta, IL-6 and TNF-alpha production is dependent on exposure to infectious virus, IFN-alpha/beta release is also induced by UV-inactivated virus. In a first step, an influenza A virus infection primes mononuclear phagocytes by leading to an accumulation of cytokine mRNA which, in a second step, is readily translated into bioactive cytokines in particular when triggering signals such as LPS are available. Therefore, influenza A virus represents an ultimately fatal macrophage activating factor which, when inducing moderate amounts of cytokines, may be beneficial by mounting an immediate antiviral response, but which causes adverse effects when cytokine release is highly elevated, for example by bacterial products because of sepsis and opportunistic infections or during drug treatment of the viral disease.

Antibody administration of AB-TIRC7 in a mouse model of collagen induced arthritis was in the past shown to provide an inhibitory effect on the progression and induction of collagen induced arthritis, in particular in combination with soluble TNF-alpha (Utku et al. 2006)

A mouse mutant deficient in the expression of TIRC7 (TIRC7 -/-) showed augmented T and B cell activation as well as cytokine response following stimulation with anti-CD40 Ab or LPS plis IL-4. Also an augmented delayed type hypersensitivity response was observed in TIRC 7 -/- mice (Utku et al, 2006).

Patent document WO 03/054018 pertains to the prevention or treatment of inflammatory diseases comprising the administration of a TIRC7 antagonist. Particularly disclosed is the prevention or treatment of arthritis.

In a mouse model of influenza infection it was shown that inflammation of airways in infected animals consisted predominantly of macrophages and lymphocytes, thus predominantly a TH1 immune response hallmarked by interferon gamma (Grunewald et al., 2001).

WO 03/025000 provides a disclosure pertaining to peptides for the inhibition of the proliferation of peripheral blood mononuclear cells (PBMCs). The peptides are derived from HLA class II alpha chain, or TIRC7 co-stimulatory molecule.

### Summary of the invention

The present invention pertains to the subject-matter of claims 1-8.

The use of the therapeutic TIRC7 based agents in accordance with the present invention may be accompanied by the use of further therapeutic agents or means of therapeutic intervention such anti-viral drugs, anti-tumor agents, chemotherapy, surgery, etc,

Hence, in one aspect, the present invention provides a drug combination of at least one TIRC7 specific agent together, or being designed to be sequentially administered, with at least one anti-viral or anti-cancer drug.

The present disclosure also concerns diagnostic methods for the detection and/or monitoring of progression of CRS including in vivo imaging techniques utilizing TIRC7 specific molecules which are preferably labeled with a detectable moiety.

In summary, the present invention provides new therapeutic and diagnostic strategies to approach CRS as characterized in the appended claims and the embodiments described below.

### Detailed description of the invention

The present disclosure relates to pharmaceutical compositions and methods for treating Cytokine Release Syndrome (CRS) in a patient, comprising administering to a patient in need thereof a therapeutically effective amount of an agent specific for TIRC7.

The present invention is based on the observation that treatment of human MDMs (monocyte derived macrophages) infected by influenza viruses with anti-TIRC7 antibodies or soluble TIRC7 ligand protein, HLA DR α chain, results in a significant inhibition of the expression of several inflammatory cytokines such as IFN-γ, IL6 and TNF-α, indicating that compounds derived from or directed against TIRC7 control hyperactivation of the immune response during viral infection and prevent the lethal outcome of the accompanying disease CRS; see the appended Example.

Thus, the finding that TIRC7 specific agents seem to have a neutralizing effect against cytokines such as IFN-γ, TNF-α, IL-2, IL-6 and MCP1 makes it prudent to stipulate that TIRC7 inhibitors/antagonists are capable of counteracting undesired concerted burst of cytokine expression/activity and, thus, are useful for the prevention, amelioration and treatment of CRS which accompanies infectious diseases, especially viral infectious diseases and hampers the therapeutic use of quite a number of drugs such as anti-viral and anti-tumor drugs as well as immunosuppressive drugs. In particular, it is known that bacterial and viral infection as well as sepsis are often accompanied by the induction of exaggerated cytokine release involving TNF-α, IFN-γ, and IL-6. Similarly, the administration of immunosuppressive drugs such as humanized monoclonal antibody CAMPATH 1-H and anti-CD3 antibody OKT3 is associated with a first-dose CRS. Furthermore, the treatment with anti-neoplastic agents such as oxaliplatin has been found to frequently induce CRS in cancer patients.

The present invention provides a novel therapeutic target in the treatment of CRS. Accordingly, the present invention discloses that TIRC7 is an important novel target for treating and diagnosing CRS evoked by and/or accompanying disorders such as bacterial and viral infection, sepsis, cancer, and drug therapy, in particular antibody based drug therapy. In one aspect, the present invention relates to an antagonist/inhibitor of T cell immune response cDNA7 (TIRC7) for the treatment or prophylaxis of cytokine release syndrome (CRS).

While previous attempts to prevent or ameliorate CRS focused on tackling one or more of the cytokines involved separately, for example by using anti-gamma interferon and anti-interleukin-6 antibodies, the present invention offers the advantage to counteract the cytokines and their expression, respectively, with one therapeutic molecule only, i.e. the TIRC7 specific agent.

The term "TIRC7" as used in accordance with the present invention denotes a protein which initially has been described to be involved in the signal transduction of T-cell activation and proliferation and that, preferably in a soluble form is capable of inhibiting or suppressing T-cell proliferation in response to alloactivation in a mixed lymphocyte culture or in response to mitogens when exogeneously added to the culture. *In vitro* translated TIRC7 protein has been shown to be able to efficiently suppress in a dose dependent manner the proliferation of T-cells in response to alloactivation in a mixed lymphocyte culture or in response to mitogens. TIRC7 is known to the person skilled in the art and described, inter alia, in international application WO99/11782, Utku, Immunity 9 (1998), 509-518 and Heinemann, Genomics 57 (1999), 398-406, which also disclose the amino and nucleic acid sequences of TIRC7. Moreover, nucleotide and amino acid sequences of TIRC7 are available in the Gene Bank database. Methods for the production of recombinant proteins and nucleic acid probes are well-known to the person skilled in the art; see, e.g., Sambrook, Molecular Cloning A Laboratory Manual, Cold Spring Harbor Laboratory (1989) N.Y. and Ausubel, Current Protocols in Molecular Biology, Green Publishing Associates and Wiley Interscience, N.Y. (1989), (1994).

TIRC7 is a cell membrane molecule expressed 24 hours after alloactivation of T-lymphocytes. Targeting of TIRC7 with polyclonal antibodies decreases IL-2 transcription, inhibits the release of the Th1-type cytokines IL-2 and IFN-γ, but does not affect the release of Th2-type cytokines such as IL-4 and IL-10, Utku, Immunity 9 (1998), 509-518. These effects result in the inhibition of inflammatory pathways and the induction of Th1-cell hypo-responsiveness to alloantigen (Utku, Immunity 9 (1998), 509-518). TIRC7 mAb has modulating effects in several immune-mediated disorders where Th1-cells are thought to play a causative role. In an animal model of rheumatoid arthritis, TIRC7 monoclonal antibodies (mAb) had both preventative and therapeutic effects; see international application WO03/054018. In experimental kidney transplantation, TIRC7 antibody prolonged organ survival and reduced graft infiltration by lymphocytes (Utku et al., 1998). Moreover, TIRC7 mAb in animal models of solid organ transplantation suppressed the migration of CD3+ lymphocytes into grafted tissue (Kumamoto et al., Am J Transplant 4 (2004), 505-514).

The terms "treatment", "treating" and the like are used herein to generally mean obtaining a desired pharmacological and/or physiological effect. The effect may be prophylactic in terms of completely or partially preventing a disease or symptom thereof and/or may be therapeutic in terms of partially or completely curing a disease and/or adverse effect attributed to the disease. The term "treatment" as used herein covers any treatment of a disease in a mammal, particularly a human, and includes: (a) preventing the disease from occurring in a subject which may be predisposed to the disease but has not yet been diagnosed as having it; (b) inhibiting the disease, i.e. arresting its development; or (c) relieving the disease, i.e. causing regression of the disease.

Furthermore, the term "subject" as employed herein relates to mammals in need of amelioration, treatment and/or prevention of CRS as disclosed herein. As used herein, the term "mammal" means any member of the higher vertebrate animals included in the class Mammalia, as defined in Webster's Medical Desk Dictionary 407 (1986), and includes but is not limited to humans, other primates, pigs, dogs, and rodents (such as immune-suppressed mice). In the preferred embodiment of this invention, the mammal is a human.

In accordance with the present invention, an agent specific for TIRC7 is preferably an antagonist/inhibitor which includes chemical agents that modulate the action of TIRC7, either through altering its enzymatic or biological activity or through modulation of expression, e.g., by affecting transcription or translation. In some cases the agent may also be a substrate or ligand binding molecule of TIRC7. Appropriate agents which bind to and/or modulate the activity TIRC7 as well as corresponding pharmaceutical and diagnostic compositions that can be adapted and used in accordance to the present invention have been described by the applicant in prior patent applications. For example, international application WO99/11782 discloses antagonistic TIRC7 proteins, peptides and polynucleotides as well as anti-TIRC7 antibodies and screening methods for identifying activators and inhibitors of TIRC7. In one embodiment of the present invention, the agent is a nucleic acid molecule comprised in the pharmaceutical composition designed for the expression of the agent by cells in vivo by, for example, direct introduction of said nucleic acid molecule or introduction of a corresponding plasmid, a plasmid in liposomes, or a viral vector (e.g. adenoviral, retroviral) containing said nucleic acid molecule. Particularly suitable vectors are described in, e.g. Fathallah-Shaykh, J. Immunol. 164 (2000), 217-222 and Varda-Bloom, Gene Therapy 8 (2001), 819-827.

In a preferred embodiment of the present invention, said agent is selected from the group consisting of:
(i) an anti-TIRC7 antibody or an anti-TIRC7-ligand antibody; or
(ii) a non-stimulatory form of TIRC7 or a soluble form of TIRC7 or TIRC7-ligand.

Examples of such agents have been described by the applicant before; see, e.g. international application WO02/36149 which discloses TIRC7 inhibitors interfering with the interaction of TIRC7 with its ligand, preferably the HLA class II α chain. Furthermore, international application WO03/025000 discloses TIRC7 antagonistic peptides, in particular TIRC7 and HLA class II a chain peptides. In another preferred embodiment, the TIRC7 ligand is derived from a natural ligand of TIRC7 such as HLA-DR alpha. Thus, the TIRC7 ligand is a HLA-DR alpha chain (HLA-DR alpha 2 protein) peptide or a fusion protein thereof such as described in international application PCT/EP2006/000560.

Preferably, one of the extracellular domains of TIRC as indicated in Fig. 1B and 2 of international application WO99/11782 is targeted, most preferably the third and largest domain between the 5^{th} and 6^{th} transmembrane region.

An anti-TIRC7 antibody to be used in accordance with pharmaceutical and diagnostic compositions of the present invention can be preferably a monoclonal antibody, but also include a polyclonal antibody, a single chain antibody, human or humanized antibody, primatized, chimerized or fragment thereof that specifically binds TIRC7 peptide or polypeptide also including bispecific antibody, synthetic antibody, antibody fragment, such as Fab, Fv or scFv fragments etc., or a chemically modified derivative of any of these. The general methodology for producing antibodies is well-known and has been described in, for example, Köhler and Milstein, Nature 256 (1975), 494 and reviewed in J.G.R. Hurrel, ed., "Monoclonal Hybridoma Antibodies: Techniques and Applications", CRC Press Inc., Boco Raron, FL (1982), as well as that taught by L. T. Mimms et al., Virology 176 (1990), 604-619. Particularly suitable therapeutic and diagnostic monoclonal anti-TIRC7 antibodies as well as combination preparations are described in international application WO03/054018 and WO03/054019. In addition, bispecific molecules that comprise at least two binding domains which *inter alia* bind TIRC7 and HLA or TCR, in particular γ-TCR or β-TCR are described in international application WO 03/091285 and WO 03/093318, respectively.

In a particular preferred embodiment of the present invention the antibody used in the pharmaceutical or diagnostic compositions, is anti-TIRC7 antibody #9 (Metiliximab©) disclosed in international application WO03/054019 or anti-TIRC7 antibody #17 (Neliximab©) disclosed in international application WO03/054018 and WO03/054019 or antibody #18 (Auriliximab©) disclosed in international application WO 03/054019.

As mentioned, the present invention is based on the surprising observation that contacting human MDMs (monocytes derived macrophages) infected by influenza viruses with monoclonal anti-TIRC7 antibodies significantly decreased the expression of several inflammatory cytokines, indicating that the modulation of immune response using compounds derived from or interacting with TIRC7 might control cytokine release during the viral infection and sepsis. It is therefore expected that TIRC7 has a potential in regulation of the lymphocytes' cytokine release in response to viral and bacterial attack. Accordingly, it is prudent to stipulate that TIRC7 and inhibitors thereof will be of benefit for modulating cytokine expression which in turn can be used for the treatment of, for example, viral infections such as influenza and avian virus infection. Most beneficially, the medical treatment in accordance with the present invention is applied to patients suffering from infection by an influenza A virus, particularly of the H5 or H7 type, and especially of the H5N1 or H5N3 type since infection by these quite aggressive influenza strains are accompanied by most severe adverse cytokine expression.

In another embodiment of the present invention, the CRS to be treated in a patient is due to side effects of a drug therapy. For example, is has been reported that oxaliplatin, a third-generation platinum analogue with proven anti-tumor activity induces CRS in colorectal cancer patients; see Tonini et al., J. Biol. Regul. Homeost. Agents 16 (2002), 105-109. Another instance has been described for high-dose cytarabine treatment in children; see Ek et al., Med. Pediatr. Oncol. 37 (2001), 459-464. Hence, the medical use of the present invention is also expected to be advantageously applied to cancer patients undergoing chemotherapy or other tumor therapy.

In a still further embodiment of the present invention, the patients to be treated in order to prevent or ameliorate CRS receive immunotherapy, in particular antibody-based immunotherapy which application is still limited by development of CRS, probably by advers effector functions of the antibodies Fc regions. One prominent example, is the humanized Orthoclone OKT 3 (mOKT3) antibody, a highly effective agent for the reversal of steroid-resistant renal allograft rejection but at risk to development of a human anti-mouse antibody response (HAMA) and by the cytokine release syndrome; see Xu et al., Cell Immunol. 200 (2000), 16-26.

Hence, in view of the above, in the pharmaceutical compositions and methods of the present invention further therapeutic agents may be used for the originally intended therapy. In particular, the TIRC7 specific agent may be accompanied by the use of an anti-viral agent such as Tamiflu©, an anti-tumor agent such as oxaliplatin or an immunosuppressive agent such as OKT3 antibody. In this aspect, the present invention also relates to pharmaceutical compositions of a drug combination of at least one TIRC7 specific agent as defined hereinbefore together, or being designed to be sequentially administered, with at least one anti-viral, anti-cancer or immunosuppressive drug. Preferably, said anti-viral drug is effective against influenza virus.

The pharmaceutical compositions described herein can be administered in a variety of different ways. Examples include administering a composition containing a pharmaceutical acceptable carrier via oral, intranasal, rectal, topical, intraperitoneal, intravenous, intramuscular, subcutaneous, subdermal, transdermal, intrathecal, and intracranial methods. Further guidance regarding formulations that are suitable for various types of administration can be found in Remington's Pharmaceutical Sciences, Mace Publishing Company, Philadelphia, PA, 17th ed. (1985) and corresponding updates. For a brief review of methods for drug delivery see Langer, Science 249 (1990), 1527-1533.

The effective amount of a therapeutic composition to be given to a particular patient will depend on a variety of factors, several of which will be different from patient to patient. A competent clinician will be able to determine an effective amount of a therapeutic agent to administer to a patient to prevent or decrease ongoing CRS. Dosage of the agent will depend on the treatment, route of administration, the nature of the therapeutics, sensitivity of the patient to the therapeutics, etc. Utilizing LDSO animal data, and other information, a clinician can determine the maximum safe dose for an individual, depending on the route of administration.

Utilizing ordinary skill, the competent clinician will be able to optimize the dosage of a particular therapeutic composition in the course of routine clinical trials. The compositions can be administered to the subject in a series of more than one administration. For therapeutic compositions, regular periodic administration will sometimes be required, or may be desirable. Therapeutic regimens will vary with the agent, e.g. a small organic compound may be taken for extended periods of time on a daily or semi-daily basis, while more selective agents, such as antagonists of TIRC7, may be administered for more defined time courses, e.g. one, two three or more days, one or more weeks, one or more months, etc., taken daily, semi-daily, semi-weekly, weekly, etc.

The present disclosure also provides a pharmaceutical pack or kit comprising one or more containers filled with one or more of the ingredients of the pharmaceutical compositions of the present invention. Associated with such container(s) can be a notice in the form prescribed by a governmental agency regulating the manufacture, use or sale of pharmaceuticals or biological products, which notice reflects approval by the agency of manufacture, use or sale for human administration.

Also disclosed is a method of diagnosing CRS as defined herein-above in a subject comprising:
(a) assaying a sample from a subject for TIRC7 transcriptional activity or TIRC7 protein; and
(b) determining the existence of CRS, wherein the induction of TIRC7 transcriptional activity or the abnormal presence or activity of TIRC7 protein compared to a control indicates the presence of CRS.

In these embodiments, the TIRC7 polynucleotides, nucleic acid molecules, (poly)peptide, antibodies or ligands preferably detectably labeled. A variety of techniques are available for labeling biomolecules, are well known to the person skilled in the art and are considered to be within the scope of the present invention. Such techniques are, e.g., described in Tijssen, "Practice and theory of enzyme immuno assays", Burden, RH and von Knippenburg (Eds), Volume 15 (1985), "Basic methods in molecular biology"; Davis LG, Dibmer MD; Battey Elsevier (1990), Mayer et al., (Eds) "Immunochemical methods in cell and molecular biology" Academic Press, London (1987), or in the series "Methods in Enzymology", Academic Press, Inc. There are many different labels and methods of labeling known to those of ordinary skill in the art. Commonly used labels comprise, inter alia, fluorochromes (like fluorescein, rhodamine, Texas Red, etc.), enzymes (like horse radish peroxidase, β-galactosidase, alkaline phosphatase), radioactive isotopes (like ³²P or ¹²⁵I), biotin, digoxygenin, colloidal metals, chemi- or bioluminescent compounds (like dioxetanes, luminol or acridiniums). Labeling procedures, like covalent coupling of enzymes or biotinyl groups, iodinations, phosphorylations, biotinylations, random priming, nick-translations, tailing (using terminal transferases) are well known in the art. Detection methods comprise, but are not limited to, autoradiography, fluorescence microscopy, direct and indirect enzymatic reactions, etc.

In addition, the above-described compounds etc. may be attached to a solid phase. Solid phases are known to those in the art and may comprise polystyrene beads, latex beads, magnetic beads, colloid metal particles, glass and/or silicon chips and surfaces, nitrocellulose strips, membranes, sheets, animal red blood cells, or red blood cell ghosts, duracytes and the walls of wells of a reaction tray, plastic tubes or other test tubes. Suitable methods of immobilizing TIRC7 nucleic acids, (poly)peptides, proteins, antibodies, etc. on solid phases include but are not limited to ionic, hydrophobic, covalent interactions and the like. The solid phase can retain one or more additional receptor(s) which has/have the ability to attract and immobilize the region as defined above. This receptor can comprise a charged substance that is oppositely charged with respect to the reagent itself or to a charged substance conjugated to the capture reagent or the receptor can be any specific binding partner which is immobilized upon (attached to) the solid phase and which is able to immobilize the reagent as defined above.
Commonly used detection assays can comprise radioisotopic or non-radioisotopic methods. These comprise, inter alia, RIA (Radioisotopic Assay) and IRMA (Immune Radioimmunometric Assay), EIA (Enzym Immuno Assay), ELISA (Enzyme Linked Immuno Assay), FIA (Fluorescent Immuno Assay), and CLIA (Chemioluminescent Immune Assay). Other detection methods that are used in the art are those that do not utilize tracer molecules. One prototype of these methods is the agglutination assay, based on the property of a given molecule to bridge at least two particles.

For diagnosis and quantification of (poly)peptides, polynucleotides, etc. in clinical and/or scientific specimens, a variety of immunological methods, as described above as well as molecular biological methods, like nucleic acid hybridization assays, PCR assays or DNA Enzyme Immunoassays (Mantero et al., Clinical Chemistry 37 (1991), 422-429) have been developed and are well known in the art. In this context, it should be noted that the TIRC7 nucleic acid molecules may also comprise PNAs, modified DNA analogs containing amide backbone linkages. Such PNAs are useful, inter alia, as probes for DNA/RNA hybridization.

The above-described compositions may be used for methods for detecting expression of a TIRC7 polynucleotide by detecting the presence of mRNA coding for a TIRC7 (poly)peptide which comprises, for example, obtaining mRNA from cells of a subject and contacting the mRNA so obtained with a probe/primer comprising a nucleic acid molecule capable of specifically hybridizing with a TIRC7 polynucleotide under suitable hybridization conditions, and detecting the presence of mRNA hybridized to the probe/primer. Further diagnostic methods leading to the detection of nucleic acid molecules in a sample comprise, e.g., polymerase chain reaction (PCR), ligase chain reaction (LCR), Southern blotting in combination with nucleic acid hybridization, comparative genome hybridization (CGH) or representative difference analysis (RDA). These methods for assaying for the presence of nucleic acid molecules are known in the art and can be carried out without any undue experimentation.

Also disclosed is a kit for determining cytokine release syndrome, said kit comprising an anti-TIRC7 antibody or TIRC7 nucleic acid probe. An appropriate TIRC7 nucleic acid probe may be for example a nucleic acid molecule of at least 15 nucleotides in length hybridizing specifically with TIRC7 mRNA. Such kits are used to detect DNA which hybridizes to TIRC7 DNA or to detect the presence of TIRC7 protein or peptide fragments in a sample. Such characterization is useful for a variety of purposes including but not limited to forensic analyses, diagnostic applications, and epidemiological studies in accordance with the above-described methods of the present invention. The recombinant TIRC7 proteins, DNA molecules, RNA molecules and antibodies lend themselves to the formulation of kits suitable for the detection and typing of TIRC7. Such a kit would typically comprise a compartmentalized carrier suitable to hold in close confinement at least one container. The carrier would further comprise reagents such as recombinant TIRC7 protein or anti-TIRC7 antibodies suitable for detecting TIRC7. The carrier may also contain a means for detection such as labeled antigen or enzyme substrates or the like.

The methods and uses as disclosed may be desirably employed in humans, although animal treatment is also encompassed by the methods and uses described herein.

These and other embodiments are disclosed and encompassed by the description and examples of the present invention. Further literature concerning any one of the materials, methods, uses and compounds to be employed in accordance with the present invention may be retrieved from public libraries and databases, using for example electronic devices. For example the public database "Medline" may be utilized, which is hosted by the National Center for Biotechnology Information and/or the National Library of Medicine at the National Institutes of Health. Further databases and web addresses, such as those of the European Bioinformatics Institute (EBI), which is part of the European Molecular Biology Laboratory (EMBL) are known to the person skilled in the art and can also be obtained using internet search engines. An overview of patent information in biotechnology and a survey of relevant sources of patent information useful for retrospective searching and for current awareness is given in Berks, TIBTECH 12 (1994), 352-364.

The above disclosure generally describes the present invention. Several documents are cited throughout the text of this specification. Full bibliographic citations may be found at the end of the specification immediately preceding the claims.

### EXAMPLES

The examples which follow further illustrate the invention, but should not be construed to limit the scope of the invention in any way. Detailed descriptions of conventional methods, such as those employed herein can be found in the cited literature; see also "The Merck Manual of Diagnosis and Therapy" Seventeenth Ed. ed by Beers and Berkow (Merck & Co., Inc. 2003).

The practice of the present invention will employ, unless otherwise indicated, conventional techniques of cell biology, cell culture, molecular biology, transgenic biology, microbiology, recombinant DNA, and immunology, which are within the skill of the art.
Methods in molecular genetics and genetic engineering are described generally in the current editions of Molecular Cloning: A Laboratory Manual, (Sambrook et al., (1989) Molecular Cloning: A Laboratory Manual, 2nd ed., Cold Spring Harbor Laboratory Press); DNA Cloning, Volumes I and II (Glover ed., 1985); Oligonucleotide Synthesis (Gait ed., 1984); Nucleic Acid Hybridization (Hames and Higgins eds. 1984); Transcription And Translation (Hames and Higgins eds. 1984); Culture Of Animal Cells (Freshney and Alan, Liss, Inc., 1987); Gene Transfer Vectors for Mammalian Cells (Miller and Calos, eds.); Current Protocols in Molecular Biology and Short Protocols in Molecular Biology, 3rd Edition (Ausubel et al., eds.); and Recombinant DNA Methodology (Wu, ed., Academic Press). Gene Transfer Vectors For Mammalian Cells (Miller and Calos, eds., 1987, Cold Spring Harbor Laboratory); Methods In Enzymology, Vols. 154 and 155 (Wu et al., eds.); Immobilized Cells And Enzymes (IRL Press, 1986); Perbal, A Practical Guide To Molecular Cloning (1984); the treatise, Methods In Enzymology (Academic Press, Inc., N.Y.); Immunochemical Methods In Cell And Molecular Biology (Mayer and Walker, eds., Academic Press, London, 1987); Handbook Of Experimental Immunology, Volumes I-IV (Weir and Blackwell, eds., 1986). Reagents, cloning vectors, and kits for genetic manipulation referred to in this disclosure are available from commercial vendors such as BioRad, Stratagene, Invitrogen, and Clontech. General techniques in cell culture and media collection are outlined in Large Scale Mammalian Cell Culture (Hu et al., Curr. Opin. Biotechnol. 8 (1997), 148); Serum-free Media (Kitano, Biotechnology 17 (1991), 73); Large Scale Mammalian Cell Culture (Curr. Opin. Biotechnol. 2 (1991), 375); and Suspension Culture of Mammalian Cells (Birch et al., Bioprocess Technol. 19 (1990), 251); Extracting information from cDNA arrays, Herzel et al., CHAOS 11, (2001), 98-107.

### Example 1: Experimental analysis of the inhibitory effects of TIRC7 ligation using antibodies or soluble ligands on infected human macrophages with avian virus

For elucidating inhibiting effects of TIRC7 ligation on infected human macrophages with avian virus, human adult blood with was collected from normal volunteers. Subsequently, human MDMs (monocytes derived macrophages) were generated from mononuclear cells. The purity of monocytes, as determined by flow cytometry (Coulter Epics Elite; Beckman Coulter) with anti-CD 14 monoclonal antibody (PharMingen) was consistently > 90%. The monocytes were refed by fresh medium every 2 days and allowed to differentiate for 14 days in vitro.

Viruses were cultured in Madin-Darby canine kidney (MDCK) cells (ATCC), were purified by adsorption to and elution from turkey red blood cells, and stored at -70°C until use. The titer of virus stock was determined by titration in MDCK cells and by daily observation for cytopathogenic effect and was confirmed by hemagglutination assay.

Day-14 differentiated MDMs were infected by influenza viruses at an MOI of 2. After virus adsorption for 1 h at 37°C, unadsorbed virus was removed by washing with PBS. Mock-treated cells were similarly treated in parallel, except that virus was not added. To determine the infectivity, cells were fixed and analyzed by immunofluorescent staining specific for influenza A virus matrix protein (M) and nucleoprotein (NP) (DAKP Imagen; Dako Diagnostics).

For performing ELISA for supernatants from virus-infected MDMs, supernatants from 10⁶ mock- or virus-infected MDMs per well incubated in a volume of 800*µ*L of RPMI 1640 plus 10% fetal bovine serum were collected after 6,12, or 24 h and stored at -70°C; thawed; and irradiated at a dosage of 0,2 J/cm² for 15 min in a UV crosslinker (Spectrolinker) before use. The concentrations of IFN-alpha, beta, gamma, TNF-alpha, MCP1, IL6, IL8 in culture supernatants were measured by use of commercially available ELISA kits purchased from R&D Systems, in accordance with the manufacturer's instructions. Each sample was assayed in duplicate. Samples were read at 450nm, using a microplate reader (Bio-Rad), and were analyzed using Microplate Manager software (version 4.0; Bio-Rad).

Analysis of the samples revealed that consistent with the findings in other studies immune responses to human influenza viruses, inflammatory cytokine response were induced by avian influenza virus in the present experiments. The treatment of the infected cell with antibodies against TIRC7 (for example Neliximab© disclosed in international application WO03/054018 and WO03/054019, see also Utku et al., Clin. Exp. Immunol. 144 (2006), 142-151 and its soluble ligand protein (for example HLA-DR alpha chain fusion protein described in international application PCT/EP2006/000560), respectively, was able to significantly inhibit the expression of several cytokines such as IFN-gamma, IL6 and TNF-alpha indicating that the modulation of immune response using compounds derived from TIRC7 might control hyperactivation of the immune response during the viral infection and prevent the lethal outcome of the disease.

## Claims

1. An antagonist / inhibitor of T-cell immune response cDNA7 (TIRC7), namely anti-TIRC7 antibody or HLA-DR alpha2 protein, for use in the treatment, prophylaxis or diagnosis of cytokine release syndrome.

2. The antagonist / inhibitor of T-cell immune response cDNA7 (TIRC7) for use in the treatment, prophylaxis or diagnosis of cytokine release syndrome according to claim 1, wherein said antagonist / inhibitor of TIRC7 is used as the only therapeutic molecule to counteract the expression of cytokines in said treatment, prophylaxis or diagnosis of cytokine release syndrome.

3. An antagonist / inhibitor of T-cell immune response cDNA7 (TIRC7) for use in the treatment, prophylaxis or diagnosis of cytokine release syndrome, according to claim 1 or 2, **wherein** the cytokine release syndrome involves one or more cytokines selected from the group consisting of IFN-Y, TNF-a, IL-2, IL-6 and MCP1.

4. An antagonist / inhibitor of T-cell immune response cDNA7 (TIRC7) for use in the treatment, prophylaxis or diagnosis of cytokine release syndrome, according to claim 3, **wherein** said cytokine release syndrome is evoked by viral infection or sepsis.

5. An antagonist / inhibitor of T-cell immune response cDNA7 (TIRC7) for use in the treatment, prophylaxis or diagnosis of cytokine release syndrome, according to claim 4, **wherein** the viral infection is caused by an influenza A virus.

6. An antagonist / inhibitor of T-cell immune response cDNA7 (TIRC7) for use in the treatment, prophylaxis or diagnosis of cytokine release syndrome, according to claim 5, **wherein** the anti-TIRC7 antibody is a monoclonal antibody.

7. An antagonist / inhibitor of T-cell immune response cDNA7 (TIRC7) for use in the treatment, prophylaxis or diagnosis of cytokine release syndrome, according to claim 6, **wherein** the anti-TIRC7 antibody or HWDR alpha2 protein is accompanied by the use of an anti-viral agent or an anti-tumor agent.

8. An antagonist / inhibitor of T-cell immune response cDNA7 (TIRC7) for use in the treatment, prophylaxis or diagnosis of cytokine release syndrome, according to claim 7, **wherein** said anti-viral drug is an anti-influenza-viral drug.

## Patentansprüche

1. Ein Antagonist / Inhibitor von T-cell immune response cDNA7 (TIRC7), nämlich ein anti-TIRC7 Antikörper oder HLA-DR Alpha2-Protein, zur Verwendung in der Behandlung, Prophylaxe oder Diagnose von Zytokinausschüttungssyndrom.

2. Der Antagonist / Inhibitor von T-cell immune response cDNA7 (TIRC7) zur Verwendung in der Behandlung, Prophylaxe oder Diagnose von Zytokinausschüttungssyndrom gemäß Anspruch 1, wobei genannter Antagonist / Inhibtor von TIRC7 in genannter Behandlung, Prophylaxe oder Diagnose des Zytokinausschüttungssyndroms als einziges therapeutisches Molekül verwendet wird, das der Expression von Zytokinen entgegenwirkt.

3. Ein Antagonist / Inhibitor von T-cell immune response cDNA7 (TIRC7) zur Verwendung in der Behandlung, Prophylaxe oder Diagnose von Zytokinausschüttungssyndrom, gemäß Anspruch 1 oder 2, wobei das Zytokinausschüttungssyndrom einhergeht mit einem oder mehreren Zytokinen ausgesucht aus der Gruppe bestehend aus IFN-Y, TNF-a, IL-2, IL-6 und MCP1.

4. Ein Antagonist / Inhibitor von T-cell immune response cDNA7 (TIRC7) zur Verwendung in der Behandlung, Prophylaxe oder der Diagnose des Zytokinausschüttungssyndroms, gemäß Anspruch 3, wobei das genannte Zytokinausschüttungssyndrom durch virale Infektion oder Sepsis hervorgerufen wird.

5. Ein Antagonist / Inhibitor von T-cell immune response cDNA7 (TIRC7) zur Verwendung in der Behandlung, Prophylaxe oder Diagnose des Zytokinausschüttungssyndroms, gemäß Anspruch 4, wobei die virale Infektion durch einen Influenza A Virus hervorgerufen wird.

6. Ein Antogonist/Inhibitor von T-cell immune response cDNA7 (TIRC7), zur Verwendung in der Behandlung, Prophylaxe oder Diagnose von Zytokinausschüttungssyndrom, gemäß Anspruch 5, wobei der anti-TIRC7 Antikörper ein monoklonaler Antikörper ist.

7. Ein Antagonist / Inhibitor von T-cell immune response cDNA7 (TIRC7), zur Verwendung in der Behandlung, Prophylaxe oder Diagnose von Zytokinausschüttungssyndrom, gemäß Anspruch 6, wobei der anti-TIRC 7 Antikörper oder HWDR Alpha2-Protein begleitet wird durch die Verwendung eines antiviralen Mittels oder eines Antitumor-Mittels.

8. Ein Antagonist / Inhibitor von T-cell immune response cDNA7 (TIRC7), zur Verwendung in der Behandlung, Prophylaxe oder Diagnose von Zytokinausschüttungssyndrom, gemäß Anspruch 7, wobei das genannte antivirale Arzneimittel ein Anti-Influenza-Virus Arzneimittel ist.

## Revendications

1. Antagoniste/inhibiteur de l'ADNc 7 à réponse immunitaire des lymphocytes T (TIRC7), à savoir l'anticorps anti-TIRC7 ou la protéine HLA-DR alpha2, pour une utilisation dans le traitement, la prophylaxie ou le diagnostic du syndrome de libération de cytokines.

2. Antagoniste/inhibiteur de l'ADNc 7 à réponse immunitaire des lymphocytes T (TIRC7) pour une utilisation dans le traitement, la prophylaxie ou le diagnostic du syndrome de libération de cytokines selon la revendication 1, dans lequel ledit antagoniste/inhibiteur de TIRC7 est utilisé comme étant la seule molécule thérapeutique pour contrecarrer l'expression de cytokines dans ledit traitement, ladite prophylaxie ou ledit diagnostic du syndrome de libération de cytokines.

3. Antagoniste/inhibiteur de l'ADNc 7 à réponse immunitaire des lymphocytes T (TIRC7) pour une utilisation dans le traitement, la prophylaxie ou le diagnostic du syndrome de libération de cytokines, selon la revendication 1 ou 2, dans lequel le syndrome de libération de cytokines implique une ou plusieurs cytokine(s) choisie(s) dans le groupe constitué d'IFN-Y, de TNF-a, d'IL-2, d'IL-6 et de MCP1.

4. Antagoniste/inhibiteur de l'ADNc 7 à réponse immunitaire des lymphocytes T (TIRC7) pour une utilisation dans le traitement, la prophylaxie ou le diagnostic du syndrome de libération de cytokines, selon la revendication 3, dans lequel ledit syndrome de libération de cytokines est induit par une infection virale ou une sepsie.

5. Antagoniste/inhibiteur de l'ADNc 7 à réponse immunitaire des lymphocytes T (TIRC7) pour une utilisation dans le traitement, la prophylaxie ou le diagnostic du syndrome de libération de cytokines, selon la revendication 4, dans lequel l'infection virale est provoquée par le virus de la grippe A.

6. Antagoniste/inhibiteur de l'ADNc 7 à réponse immunitaire des lymphocytes T (TIRC7) pour une utilisation dans le traitement, la prophylaxie ou le diagnostic du syndrome de libération de cytokines, selon la revendication 5, dans lequel l'anticorps anti-TIRC7 est un anticorps monoclonal.

7. Antagoniste/inhibiteur de l'ADNc 7 à réponse immunitaire des lymphocytes T (TIRC7) pour une utilisation dans le traitement, la prophylaxie ou le diagnostic du syndrome de libération de cytokines, selon la revendication 6, dans lequel l'anticorps anti-TIRC7 ou la protéine HWDR alpha2 est accompagné(e) de l'utilisation d'un agent antiviral ou un agent anti-tumoral.

8. Antagoniste/inhibiteur de l'ADNc 7 à réponse immunitaire des lymphocytes T (TIRC7) pour une utilisation dans le traitement, la prophylaxie ou le diagnostic du syndrome de libération de cytokines, selon la revendication 7, dans lequel ledit médicament antiviral est un médicament anti-virus de la grippe.
